# EUROPEAN PATENT APPLICATION

(11) **EP 4 160 508 A1**
(43) Date of publication of application: **05.04.2023**
(21) Application number: 21811938.6
(22) Date of filing: 08.05.2021
(51) Int. Cl.: G06Q 30/06

(54) **METHOD AND APPARATUS FOR PUSHING INFORMATION**

(30) Priority: 29.05.2020 CN 202010474571
(71) Applicant: BEIJING JINGDONG TUOXIAN TECHNOLOGY CO., LTD., Beijing 100176 (CN)
(72) Inventor: TONG, Yawei, Beijing 100176 (CN); MA, Shuangliang, Beijing 100176 (CN); ZHANG, Liyao, Beijing 100176 (CN); TAO, Cong, Beijing 100176 (CN); BIAN, Meng, Beijing 100176 (CN)
(74) Representative: Mewburn Ellis LLP
(86) International application number: PCT/CN2021/092251
(87) International publication number: WO 2021/238611

(57) **Abstract**

Disclosed in embodiments of the present application are a method and apparatus for pushing information. One specific embodiment of the method comprises: obtaining a prescription image in which prescription information is present; recognizing the prescription image to obtain a recognition result; inputting the recognition result into a pre-trained prescription image analysis model to obtain the prescription information present in the prescription image; and pushing information on the basis of the prescription information. This embodiment implements a mode that a user can dominate extraction of prescription data.

## Description

### CROSS-REFERENCE TO RELATED APPLICATIONS

The present disclosure claims priority to Chinese Patent Application No. 202010474571.4, titled "METHOD AND APPARATUS FOR PUSHING INFORMATION" filed on 29 May, 2020, the full text of which is incorporated herein by reference.

### TECHNICAL FIELD

Embodiments of the present disclosure relate to the technical field of computers, and specifically relate to a method and an apparatus for pushing information.

### BACKGROUND

A prescription usually refers to a medical document that is issued by a registered licensed physician or a licensed assistant physician for a patient during diagnosis and treatment, which is audited, dispensed, checked by a skilled person specialized in pharmacy and having a qualification for professional and technical posts in pharmacy, and is used as medication evidence for the patient. The prescription is a written document based on which a doctor medicates a patient, and is the basis for a pharmacist to dispense drugs.

Extraction of prescription data is a core path and premise of prescription outflow (i.e., the flow of a prescription issued in a hospital to pharmacies, drugstores, and online drug sales platforms outside the hospital) . Relevant extracting methods of prescription data are usually hospital information system transformation and data interchange, i.e., transforming a current hospital information system such that the hospital information system supports the inquiry and extraction of prescription data from an out-of-hospital system, and supports synchronization of prescription data to the out-of-hospital system. The out-of-hospital system may sell the prescription after it obtains the prescription.

### SUMMARY

Embodiments of the present disclosure present a method and an apparatus for pushing information.

In a first aspect, an embodiment of the present disclosure provides a method for pushing information, including: obtaining a prescription image presenting prescription information; recognizing the prescription image to obtain a recognition result; inputting the recognition result into a pre-trained prescription image analyzing model to obtain the prescription information presented in the prescription image; and pushing information based on the prescription information.

In some embodiments, the pushing information based on the prescription information includes: verifying the prescription information; and pushing the information based on a verification result.

In some embodiments, the pushing the information based on the verification result includes: pushing drug purchase information based on drug information in the prescription information when the verification result indicates that the prescription information passes a verification.

In some embodiments, the pushing the information based on the verification result includes: pushing abnormal prompt information when the verification result indicates that the prescription information fails to pass the verification.

In some embodiments, the pushing the drug purchase information based on the drug information in the prescription information includes: obtaining positioning information of a user terminal, the prescription image being obtained from the user terminal; determining at least one drugstore within a preset range based on the positioning information; selecting a target drugstore from the at least one drugstore based on a preset credit rating and a position of the at least one drugstore, and based on an inventory amount and a price of a drug indicated by the drug information in the at least one drugstore; and pushing the drug purchase information, where the drug purchase information includes a drugstore identifier of the target drugstore.

In some embodiments, the drug information includes a drug price; and the selecting the target drugstore from the at least one drugstore based on the preset credit rating and the position of at least one drugstore, and based on the inventory amount and the price of the drug indicated by the drug information in the at least one drugstore includes: for each drugstore of the at least one drugstore: finding a preset weight corresponding to a preset credit rating of the drugstore as a first weight, determining a ratio of the drug price to the price of the drug indicated by the drug information in the drugstore as a second weight, finding a preset weight corresponding to the inventory amount of the drug indicated by the drug information in the drugstore as a third weight, determining a distance between the drugstore and a user using the user terminal based on the positioning information of the user terminal and a position of the drugstore, determining a fourth weight based on the distance, and determining a product of the first weight, the second weight, the third weight, and the fourth weight as a score of the drugstore; and selecting the target drugstore from the at least one drugstore based on the score of each drugstore.

In some embodiments, the determining the fourth weight based on the distance includes: finding a preset delivery duration corresponding to the distance, and determining a ratio of a preset value to the delivery duration as the fourth weight.

In some embodiments, the verifying the prescription information includes: determining whether the prescription information includes a target text; and the pushing the abnormal prompt information when the verification result indicates that the prescription information fails to pass the verification includes: pushing first abnormal prompt information when determining that the prescription information does not include the target text, where the first abnormal prompt information is configured to indicate that a prescription indicated by the prescription image is false.

In some embodiments, the verifying the prescription information includes: determining whether the prescription information does not include a target text in a target text set or a corresponding text of the target text; and the pushing the abnormal prompt information when the verifying result indicates that the prescription information fails to pass the verification includes: pushing second abnormal prompt information when determining that the prescription information does not include the target text in the target text set or does not include the corresponding text of the target text, where the second abnormal prompt information is configured to indicate that a prescription indicated by the prescription image is incomplete.

In some embodiments, the verifying the prescription information includes: extracting time-related information from the prescription information, where the time-related information includes a prescription issuing time and a prescription validity duration; determining a time difference between a current time and the prescription issuing time, and determining whether the time difference is greater than the prescription validity duration; and the pushing the abnormal prompt information when the verifying result indicates that the prescription information fails to pass the verification includes: pushing third abnormal prompt information when determining that the time difference is greater than the prescription validity duration, where the third abnormal prompt information is configured to indicate that a prescription indicated by the prescription image is expired.

In some embodiments, the verifying the prescription information includes: determining whether a prescription corresponding to the prescription information is present in a used prescription set; and the pushing the abnormal prompt information when the verifying result indicates that the prescription information fails to pass the verification includes: pushing fourth abnormal prompt information if determining that the prescription corresponding to the prescription information is present in the used prescription set, where the fourth abnormal prompt information is configured to indicate that a prescription indicated by the prescription image is used.

In some embodiments, the recognition result includes position information configured to indicate a position of a character in the prescription image; and the prescription image analyzing model is obtained by training including: obtaining a training sample set, where a training sample includes a sample recognition result and sample prescription information, the sample recognition result is recognized from a sample prescription image, and the sample prescription information is determined based on a position of each character of a corresponding sample recognition result in the sample prescription image; and training the prescription image analyzing model by taking sample recognition results of training samples in the training sample set as an input, and by taking sample prescription information corresponding to inputted sample recognition results as an expected output.

In a second aspect, an embodiment of the present disclosure provides an apparatus for pushing information, including: an obtaining unit configured to obtain a prescription image presenting prescription information; a recognizing unit configured to recognize the prescription image to obtain a recognition result; an input unit configured to input the recognition result into a pre-trained prescription image analyzing model to obtain the prescription information presented in the prescription image; and a pushing unit configured to push information based on the prescription information.

In a third aspect, an embodiment of the present disclosure provides an electronic device, including: one or more processors; and a storage apparatus, storing one or more programs thereon, where the one or more programs, when executed by the one or more processors, cause the one or more processors to implement the method according to any one implementation in the first aspect.

In a fourth aspect, an embodiment of the present disclosure provides a computer readable medium, storing a computer program thereon, where the computer program, when executed by a processor, implements the method according to any one implementation in the first aspect.

The method and the apparatus for pushing information provided in the above embodiments of the present disclosure obtain a prescription image presenting prescription information; then recognize the prescription image to obtain a recognition result; then input the recognition result into a pre-trained prescription image analyzing model to obtain the prescription information presented in the prescription image; and finally push information based on the prescription information, thereby solving the problems of great transformation difficulties and low reusability of the prescription extracting system used to obtain a prescription from a hospital information system, and implementing a mode that a user can dominate extraction of prescription data.

### BRIEF DESCRIPTION OF THE DRAWINGS

After reading detailed description of non-limiting embodiments with reference to the following accompanying drawings, other features, objectives and advantages of the present disclosure will become more apparent.
Fig. 1 is an example system architecture in which embodiments of the present disclosure may be implemented;
Fig. 2 is a flowchart of a method for pushing information according to an embodiment of the present disclosure;
Fig. 3 is a flowchart of the method for pushing information according to another embodiment of the present disclosure;
Fig. 4 is a schematic diagram of an application scenario of the method for pushing information according to the present disclosure;
Fig. 5 is a flowchart of the method for pushing information according to still another embodiment of the present disclosure;
Fig. 6 is a schematic structural diagram of an apparatus for pushing information according to an embodiment of the present disclosure; and
Fig. 7 is a schematic structural diagram adapted to implement a computer system of an electronic device according to embodiments of the present disclosure.

### DETAILED DESCRIPTION OF EMBODIMENTS

The present disclosure will be further described in detail below in combination with the accompanying drawings and the embodiments. It should be understood that the specific embodiments described here are merely used for explaining the relevant disclosure, rather than limiting the disclosure. In addition, it should be further noted that, for ease of description, only the portions related to the relevant disclosure are shown in the drawings.

It should be noted that the embodiments in the present disclosure and the features in the embodiments may be combined with each other on a non-conflict basis. The present disclosure will be described in detail below with reference to the drawings and in combination with the embodiments.

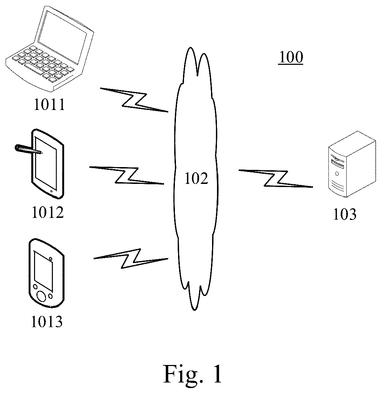

Fig. 1 shows an example system architecture 100 in which a method for pushing information according to embodiments of the present disclosure may be implemented.

As shown in Fig. 1, the system architecture 100 may include user terminals 1011, 1012 and 1013, a network 102, and a server 103. The network 102 serves as a medium providing a communication link between the user terminals 1011, 1012, and 1013, and the server 103. The network 102 may include various types of connections, such as wired or wireless communication links, or optical cables.

A user may interact with the server 103 using the user terminals 1011, 1012, and 1013 via the network 102, e.g., to send or receive a message (e.g., the server 103 may obtain a prescription image from the user terminals 1011, 1012, and 1013, and the user terminals 1011, 1012, and 1013 may also receive a message pushed by the server 103). The user terminals 1011, 1012, and 1013 may be provided with various communication client applications, such as a medical service application, a shopping application, a search application, and instant messaging software.

The user terminals 1011, 1012, and 1013 may be hardware, or may be software. When the user terminals 1011, 1012, and 1013 are hardware, the user terminals may be various electronic devices that support information interaction, including but not limited to a smart phone, a tablet computer, a laptop portable computer, a desktop computer, and the like. When the user terminals 1011, 1012, and 1013 are software, the user terminals may be installed in the above-listed electronic devices, or may be implemented as a plurality of software programs or software modules, or may be implemented as a single software program or software module. This is not specifically limited here.

The server 103 may be a server that provides various services, for example, may be a backend server that analyzes a prescription image. The server 103 may first obtain a prescription image presenting prescription information; then may recognize the prescription image to obtain a recognition result; then may input the recognition result into a pre-trained prescription image analyzing model to obtain the prescription information presented in the prescription image; and finally may push the information to the user terminals 1011, 1012, and 1013 based on the prescription information.

It should be noted that the server 103 may be hardware, or may be software. When the server 103 is hardware, the server may be implemented as a distributed server cluster composed of a plurality of servers, or may be implemented as a single server. When the server 103 is software, the server may be implemented as a plurality of software programs or software modules (e.g., software programs or software modules for providing distributed services), or may be implemented as a single software program or software module. This is not specifically limited here.

It should be noted that the method for pushing information provided in the embodiment of the present disclosure is generally executed by the server 103.

It should be understood that the numbers of terminal devices, networks, and servers in Fig. 1 are merely illustrative. Any number of terminal devices, networks, and servers may be provided based on actual requirements.

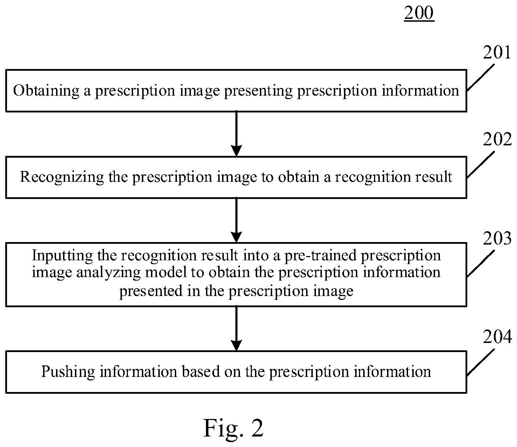

Further referring to Fig. 2, Fig. 2 illustrates a flowchart 200 of a method for pushing information according to an embodiment of the present disclosure. The method for pushing information includes the following steps.

Step 201 includes: obtaining a prescription image presenting prescription information.

In the present embodiment, an executing body (e.g., the server shown in Fig. 1) of the method for pushing information may obtain the prescription image presenting the prescription information. Here, the prescription usually includes a superscription, an inscription, and a subscription. The superscription usually includes full name of a hospital, department name, patient name, patient gender, patient age, date, and the like. Items with special requirements may be added. Prescriptions for stupefacients and first-class psychoactive drugs should further include an identity certificate number of a patient, a name of an agent, and an identity certificate number of the agent. The inscription includes a prescription head and a main portion of the prescription. The prescription head usually begins with "R" or "RP," which means to take the following drugs. The main portion of the prescription usually includes name, dosage form, specification, amount, usage, and the like of the drugs. The subscription usually includes signatures of a doctor, a pharmacist, and a biller to show their responsibility. The above prescription information may be specific information denoted in the prescription.

In the present embodiment, a user may photograph a paper prescription or select a prescription image in a local album using a user terminal, and then upload the prescription image to the executing body.

Step 202 includes recognizing the prescription image to obtain a recognition result.

In the present embodiment, the executing body may recognize the prescription image obtained in step 201 to obtain the recognition result. As an example, the executing body may recognize a character of the prescription image as the recognition result using an optical character recognition (OCR) technology. The optical character recognition technology refers to a process of checking a character printed on paper, determining a shape the character by detecting dark and bright patterns, and then converting the shape into a computer character using a character recognition method.

Step 203 includes inputting the recognition result into a pre-trained prescription image analyzing model to obtain the prescription information presented in the prescription image.

In the present embodiment, the executing body may input the recognition result recognized in step 202 into the pre-trained prescription image analyzing model to obtain the prescription information presented in the prescription image. The prescription image analyzing model may be configured to indicate a corresponding relationship between the recognition result recognized from the image and the prescription information presented in the image.

Step 204 includes pushing information based on the prescription information.

In the present embodiment, the executing body may push information to the user terminal, based on the prescription information, where the prescription image is obtained from the user terminal. As an example, since the prescription information may include a drug name and a drug amount, the executing body may select, from a preset candidate drugstore set, a drugstore whose inventory amount of a drug indicated by the drug name is greater than or equal to the drug amount, and then may push drugstore information of the selected drugstore to the user terminal. The drugstore information may include a drugstore name and a drugstore address. Drugstores in the candidate drugstore set may be drugstores in a region where a user uploading the prescription image is located.

In the present embodiment, the executing body may push the information to the user terminal, where the prescription image is obtained from the user terminal, and the user terminal may present the information after receiving the information pushed by the executing body.

In some alternative implementations of the present embodiment, the executing body may push information based on the prescription information, which includes the following steps: the executing body may verify the prescription information, e.g., may verify the authenticity and validity of the prescription information, and then may push the information based on the verification result. As an example, when the verification result indicates that a prescription indicated by the prescription image is real and valid, the drugstore information of the selected drugstore may be pushed.

In some alternative implementations of the present embodiment, the recognition result may include position information used to indicate a position of the character in the prescription image. A direction of the character may be determined based on a position of each character in the prescription image. Here, the position information may be represented by using coordinates in a preset coordinate system. The coordinate system may use any point of the prescription image (for example, a vertex of the prescription image) as the origin of coordinates, and use straight lines parallel to two adjoining sides of the prescription image as the abscissa axis and the ordinate axis of the coordinate system. The prescription image analyzing model may be obtained by training, which includes the following steps. An executing body for training the prescription image analyzing model may first obtain a training sample set. A training sample in the training sample set generally includes a sample recognition result and sample prescription information. The sample recognition result is generally recognized from the sample prescription image. The sample prescription information is generally determined based on the position of each character of the corresponding sample recognition result in the sample prescription image. Specifically, a feature text may be selected from the sample prescription image. The feature text may be manually annotated by the pharmacist, for example, may include hospital, clinic, health station, name, age, gender, Rp, doctor, physician, and date. Then, the sample prescription information may be obtained based on analysis of the feature text. For each feature text among a plurality of feature texts, coordinates of the feature text (for example, coordinates of a first character) may be used as a starting point, a character string with a closest distance from the feature text may be determined based on a vector of a text direction (character read or write direction of the feature text), and the character string may be determined as prescription information corresponding to the feature text. Then, the prescription image analyzing model may be obtained by training by taking sample recognition results of training samples in the training sample set as an input, and by taking sample prescription information corresponding to inputted sample recognition results as an expected output.

The method provided in the above embodiments of the present disclosure recognizes and analyzes an obtained prescription image to obtain prescription information, and pushes information based on the prescription information, thereby solving the problems of great transformation difficulties and low reusability of the current prescription extracting system configured to obtain a prescription from a hospital information system, and implementing a mode that a user can dominate extraction of prescription data.

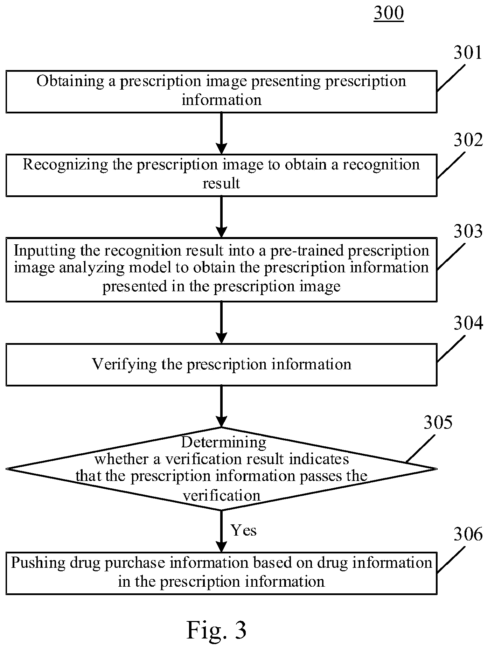

Further referring to Fig. 3, a flowchart 300 of the method for pushing information according to another embodiment of the present disclosure is shown.

Step 301 includes obtaining a prescription image presenting prescription information.

Step 302 includes recognizing the prescription image to obtain a recognition result.

Step 303 includes inputting the recognition result into a pre-trained prescription image analyzing model to obtain the prescription information presented in the prescription image.

In the present embodiment, steps 301-303 may be executed in a way similar to the way of executing steps 201-203. The description will not be repeated here.

Step 304 includes verifying the prescription information.

In the present embodiment, an executing body (e.g., the server shown in Fig. 1) of the method for pushing information may obtain a name of a physician and/or a name of a pharmacist from the prescription information. Whether the name of the physician in the prescription information is present in a pre-stored name list of registered licensed physicians and licensed assistant physicians may be determined by the executing body, or whether the name of the pharmacist in the prescription information is present in a pre-stored name list of skilled persons specialized in pharmacy and having qualifications for professional and technical posts in pharmacy may be determined by the executing body.

If the name of the physician in the prescription information is not present in the name list of registered licensed physicians and licensed assistant physicians, or the name of the pharmacist in the prescription information is not present in the name list of skilled persons specialized in pharmacy and having qualifications for professional and technical posts in pharmacy, a verification result indicating that the prescription information fails to pass the verification may be generated.

If the name of the physician in the prescription information is present in the name list of registered licensed physicians and licensed assistant physicians, and the name of the pharmacist in the prescription information is also present in the name list of skilled persons specialized in pharmacy and having qualifications for professional and technical posts in pharmacy, a verification result indicating that the prescription information passes the verification may be generated.

Step 305 includes determining whether a verification result indicates that the prescription information passes the verification.

In the present embodiment, the executing body may determine whether the obtained verification result indicates that the prescription information passes the verification. If the verification result indicates that the prescription information passes the verification, the executing body may execute step 306.

As an example, a verification result of "1" or "T" may be used to indicate that the prescription information passes the verification; and a verification result of "0" or "F" may be used to indicate that the prescription information fails to pass the verification. In this case, if the verification result is determined to be "1" or "T," the verification result may be determined to indicate that the prescription information passes the verification. If the verification result is determined to be "0" or "F," the verification result may be determined to indicate that the prescription information fails to pass the verification.

Step 306 includes pushing drug purchase information based on drug information in the prescription information if the verification result indicates that the prescription information passes the verification.

In the present embodiment, if the verification result is determined in step 305 to indicate that the prescription information passes the verification, the executing body may push the drug purchase information based on the drug information in the prescription information. Specifically, the drug information may include a drug name, a drug specification, and a drug amount. The executing body may match the drug name and the drug specification with information of each drug in a preset drug set, to find a drug indicated in the prescription image as a target drug; then may search for at least one drugstore whose inventory amount of the target drug is greater than or equal to the drug amount; then may, for each drugstore among the found at least one drugstore, generate drug purchase information that includes the drug name, a drug brand, the drug specification, and a drugstore identifier (drugstore name) of the drugstore; and finally may push the generated at least one piece of drug purchase information. It should be noted that each piece of drug purchase information among the at least one piece of drug purchase information generally corresponds to a drugstore. Here, the drug purchase information may include a drug purchase link, and a user may usually click a drug purchase link presented on a user terminal, thereby purchasing a drug indicated by the prescription image.

In some alternative implementations of the present embodiment, the executing body may push the drug purchase information based on the drug information in the prescription information, which includes the following steps: the executing body may first obtain positioning information of a user terminal, where the prescription image is obtained from the user terminal, and then may determine at least one drugstore within a preset range based on the positioning information. The preset range may be a given city, i.e., the drugstore and a user uploading the prescription image are in the given city; or the preset range may be a preset distance, for example, two kilometers. Then, the executing body may select a target drugstore from the at least one drugstore based on a preset credit rating and a position of the at least one drugstore, and an inventory amount and a price of a drug indicated by the drug information in the drugstore. Specifically, the credit rating may be represented as a star, which may be generated based on user evaluations, and may include five stars, four stars, three stars, two stars, and one star. The executing body may first select drugstores from the at least one drugstore whose inventory amount of the drug indicated by the drug information is greater than or equal to the drug amount in the drug information as first drugstores; then may select, in an ascending order of sale prices of the drug indicated by the drug information, a preset first number of drugstores from the first drugstores as second drugstores; then may determine distances between the user terminal and the second drugstores based on the positioning information of the user terminal and positions of the second drugstores, to select, in an ascending order of distances, a preset second number of drugstores from the second drugstores as third drugstores; and finally may select a drugstore with a highest credit rating from the third drugstores as the target drugstore. The executing body may push drug purchase information including a drugstore identifier of the target drugstore to the user terminal, where the prescription image is obtained from the user terminal. In this way, the target drugstore may be selected based on a plurality of dimensions, and the drugstore name of the target drugstore may be pushed to the user, thereby improving the abundance of the drug purchase information.

In some alternative implementations of the present embodiment, the drug information in the prescription information may include a drug price, i.e., a prescription may include a drug price of the drug. The executing body may select the target drugstore from the at least one drugstore based on the preset credit rating and the position of the at least one drugstore, and based on the inventory amount and the price of the drug indicated by the drug information in the drugstore, which includes the following steps: the executing body may find, for each drugstore of the at least one drugstore, a preset weight corresponding to the preset credit rating of the drugstore as a first weight. Here, the executing body may store a corresponding relationship between the credit rating and the preset weight. For example, a weight of a five-star drugstore may be 1, a weight of a four-star drugstore may be 0.8, and a weight of a three-star drugstore may be 0.6. Under normal conditions, the lower the credit rating is, the smaller the weight is. Then, the executing body may determine a ratio of the drug price in the prescription to a price of the drug indicated by the drug information in the drugstore as a second weight. As an example, if the drug price in the prescription is CNY 36, and the price of the drug in the drugstore is CNY 25, the second weight may be 1.44. Here, an upper limit of the second weight may be set, for example, as 2. It should be noted that if the prescription includes at least two drug names, the executing body may determine a ratio of a drug price of each drug in the prescription to a price of the drug in the drugstore respectively, and may determine a largest ratio as the second weight. Then, the executing body may find a preset weight corresponding to the inventory amount of the drug indicated by the drug information in the drugstore as a third weight. Here, if the inventory amount is greater than or equal to the drug amount in the prescription, the third weight may be 1; while if the inventory amount is less than the drug amount in the prescription, the third weight may be 0. Then, the executing body may determine a distance between the drugstore and a user using the user terminal based on the positioning information of the user terminal and a position of the drugstore, and determine a fourth weight based on the distance. Specifically, the executing body may store a corresponding relationship table of a corresponding relationship between the preset distance and the fourth weight, and the executing body may search for the fourth weight corresponding to the determined distance from the corresponding relationship table. Then, the executing body may determine a product of the first weight, the second weight, the third weight, and the fourth weight as a score of the drugstore. Finally, the executing body may select the target drugstore from the at least one drugstore based on the score of each drugstore. As an example, a drugstore with a highest score may be selected from the at least one drugstore as the target drugstore.

In some alternative implementations of the present embodiment, the executing body may determine the fourth weight based on the distance, which includes the following steps: the executing body may find a preset delivery duration corresponding to the distance. The executing body may store a corresponding relationship table of a corresponding relationship between the preset distance and the delivery duration, and may search a delivery duration corresponding to the distance from the corresponding relationship table. For example, a delivery duration corresponding to a distance of 500 kilometers may be found to be 2 days. Here, the delivery duration is usually measured in days. Then, a ratio of a preset value to the delivery duration may be determined as the fourth weight. The preset value may be 1. As an example, if the delivery duration is 2 days, the fourth weight may be 0.5. Here, an upper limit of the fourth weight may be set, for example, as 2.

As can be seen from Fig. 3, compared with the corresponding embodiment of Fig. 2, the flowchart 300 of the method for pushing information in the present embodiment reflects the steps of verifying prescription information, and pushing drug purchase information based on drug information in the prescription information if the prescription information passes the verification. Therefore, the solution described in the present embodiment provides a way for a user to complete drug purchase on the Internet based on prescription data, such that the user can more conveniently and quickly purchase prescription drugs.

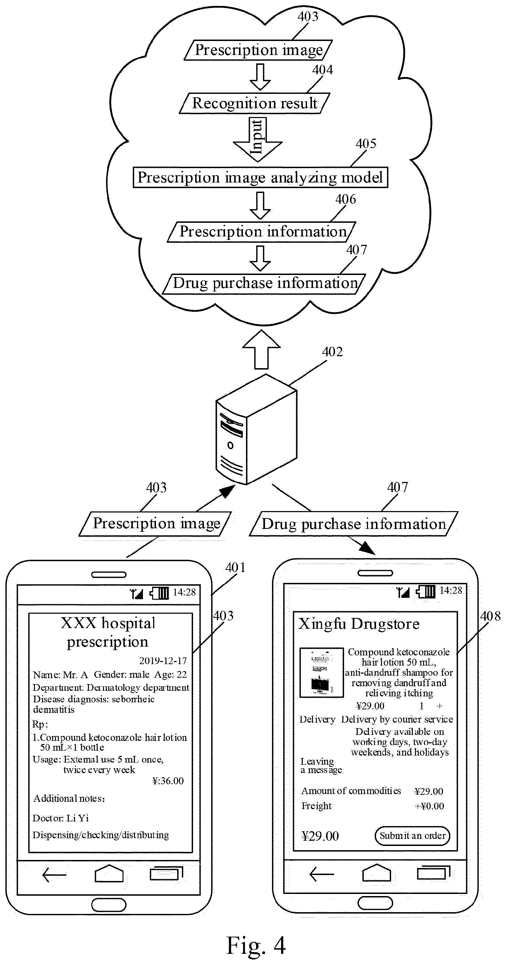

Further referring to Fig. 4, Fig. 4 is a schematic diagram of an application scenario of the method for pushing information according to the present embodiment. In the application scenario of Fig. 4, a user may photograph a paper prescription using a terminal device 401 to obtain a prescription image, as shown by an icon 403. Then, the terminal device 401 may send a prescription image 403 to a server 402, and the server 402 may recognize the received prescription image 403 to obtain a recognition result 404. The recognition result 404 may be a character included in the prescription image 403. Then, the server 402 may input the recognition result 404 into a pre-trained prescription image analyzing model 405 to obtain prescription information 406 presented in the prescription image 403. Here, the prescription information 406 may include: name: Mr. A; gender: male; age: 22 years old; department: dermatology department; disease diagnosis: seborrheic dermatitis; Rp: compound ketoconazole hair lotion, 50 mL, 1 bottle, usage: external use, 5 mL once, twice every week; price: CNY 36.00; physician: Yi Li. Then, the server 402 may generate drug purchase information 407 based on the prescription information 406. Specifically, the server 402 may match the drug name "compound ketoconazole hair lotion" and the drug specification "50 mL" with information of each drug in a preset drug set, to find a drug indicated in the prescription image 403 as a target drug; and then may search at least one drugstore with an inventory amount for the target drug greater than or equal to the drug amount of 1 bottle. Here, "Xingfu Drugstore" is found to sell the compound ketoconazole hair lotion of brand A, and the drug purchase information 407 including a drug name, a drug specification, a drug amount, and a drugstore name may be generated. Finally, the server 402 may send the drug purchase information 407 to the terminal device 401, and the terminal device may present the drug purchase information 407. In a presentation page shown by an icon 408, if the user performs a click operation on an icon "submitting an order", a drug in the prescription may be purchased.

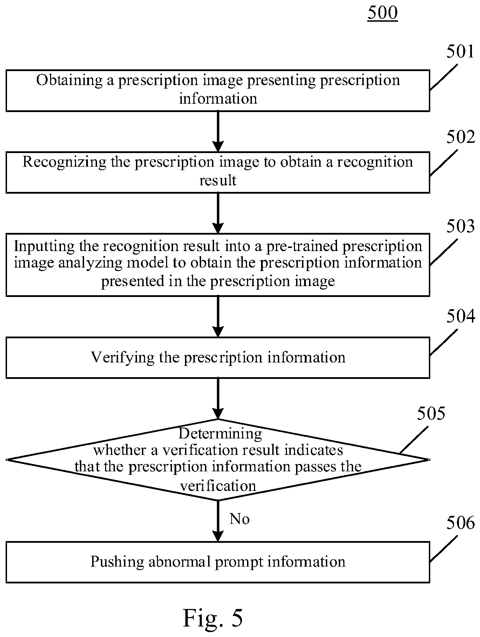

Further referring to Fig. 5, a flowchart 500 of the method for pushing information according to another embodiment of the present disclosure is shown, including the following steps.

Step 501 includes obtaining a prescription image presenting prescription information.

Step 502 includes recognizing the prescription image to obtain a recognition result.

Step 503 includes inputting the recognition result into a pre-trained prescription image analyzing model to obtain the prescription information presented in the prescription image.

In the present embodiment, steps 501-503 may be executed in a way similar to the way of executing steps 201-203. The description will not be repeated here.

Step 504 includes verifying the prescription information.

In the present embodiment, an executing body (e.g., the server shown in Fig. 1) of the method for pushing information may obtain a name of a physician and/or a name of a pharmacist from the prescription information. Whether the name of the physician in the prescription information is present in a pre-stored name list of registered licensed physicians and licensed assistant physicians may be determined by the executing body, or whether the name of the pharmacist in the prescription information is present in a pre-stored name list of skilled persons specialized in pharmacy and having qualifications for professional and technical posts in pharmacy may be determined by the executing body.

If the name of the physician in the prescription information is not present in the name list of registered licensed physicians and licensed assistant physicians, or the name of the pharmacist in the prescription information is not present in the name list of skilled persons specialized in pharmacy and having qualifications for professional and technical posts in pharmacy, a verification result indicating that the prescription information fails to pass the verification may be generated.

If the name of the physician in the prescription information is present in the name list of registered licensed physicians and licensed assistant physicians, and the name of the pharmacist in the prescription information is also present in the name list of skilled persons specialized in pharmacy and having qualifications for professional and technical posts in pharmacy, a verification result indicating that the prescription information passes the verification may be generated.

Step 505 includes determining whether a verification result indicates that the prescription information passes the verification.

In the present embodiment, the executing body may determine whether the obtained verification result indicates that the prescription information passes the verification. If the verification result indicates that the prescription information passes the verification, the executing body may execute step 506.

As an example, a verification result of "1" or "T" may be used to indicate that the prescription information passes the verification; and a verification result of "0" or "F" may be used to indicate that the prescription information fails to pass the verification. In this case, if the verification result is determined to be "1" or "T," the verification result may be determined to indicate that the prescription information passes the verification. If the verification result is determined to be "0" or "F," the verification result may be determined to indicate that the prescription information fails to pass the verification.

Step 506 includes pushing abnormal prompt information when the verification result indicates that the prescription information fails to pass the verification.

In the present embodiment, if the verification result is determined in step 505 to indicate that the prescription information fails to pass the verification, the executing body may push the abnormal prompt information. The abnormal prompt information may be used to indicate that the prescription information is abnormal. As an example, the abnormal prompt information may be "your prescription is abnormal. Please re-upload a prescription image."

In some alternative implementations of the present embodiment, the executing body may verify the prescription information through the following steps: the executing body may determine whether the prescription information includes a target text. The target text is usually a preset text recorded in a normal prescription, and usually may include a patient name, a patient age, date, a drug name, a drug specification, and a drug amount. If the prescription information is determined not to include any text in the target text, the executing body may push first abnormal prompt information. Here, the first abnormal prompt information may be used for indicating that a prescription indicated by the prescription image is false. As an example, the first abnormal prompt information may be "your prescription is false. Please re-upload a prescription image." In this way, the authenticity of the prescription can be guaranteed.

In some alternative implementations of the present embodiment, the executing body may verify the prescription information through the following steps: the executing body may determine whether the prescription information does not include all target texts in a target text set or does not include corresponding texts of all target texts. The target text is usually a preset text to be recorded in a normal prescription, and usually may include a patient name, a patient age, date, a drug name, a drug specification, and a drug amount. The corresponding text of the target text is usually a value of the target text. For example, if the target text is a patient name, the corresponding text may be Mr. A; while if the target text is a drug name, the corresponding text may be Banlangen granule. If the prescription information is determined not to include all target texts in the target text set or does not include the corresponding texts of all target texts, the executing body may push second abnormal prompt information. Here, the second abnormal prompt information may be used for indicating that a prescription indicated by the prescription image is incomplete. As an example, the second piece of abnormal prompt information may be "your prescription is incomplete. Please re-upload a prescription image." In this way, the completeness of the prescription can be guaranteed.

In some alternative implementations of the present embodiment, the executing body may verify the prescription information through the following steps: the executing body may extract time-related information from the prescription information. The time-related information may include prescription issue time and a prescription validity duration. As an example, if the prescription is annotated with that: the prescription is valid on the current day, the prescription validity duration being less than one day may be extracted; while if the prescription is annotated with that: the prescription is valid within three days, the prescription validity duration being three days may be extracted. The executing body may determine a time difference between current time and the prescription issue time, and determine whether the time difference is greater than the prescription validity duration. As an example, if the current time is December 30, 2019, the prescription issuing time is December 26, 2019, and the prescription validity duration is three days, the time difference between the current time of December 30, 2019 and the prescription issue time of December 26, 2019 may be determined to be four days, which is greater than the prescription validity duration of three days. If the time difference is determined to be greater than the prescription validity duration, the executing body may push third abnormal prompt information. Here, the third abnormal prompt information may be used for indicating that a prescription indicated by the prescription image is expired. As an example, the third abnormal prompt information may be "your prescription is expired." In this way, the timeliness of the prescription can be guaranteed.

In some alternative implementations of the present embodiment, the executing body may verify the prescription information through the following step: the executing body may determine whether the prescription corresponding to the prescription information is present in a used prescription set. Here, the executing body may store the used prescription set, and prescriptions stored in the used prescription set are usually prescriptions that have been used by a user to buy a drug. If the prescription corresponding to the prescription information is present in the used prescription set, the executing body may push fourth abnormal prompt information. Here, the fourth abnormal prompt information may be used for indicating that a prescription indicated by the prescription image is used. As an example, the fourth abnormal prompt information may be "your prescription is employed." In this way, the uniqueness of the prescription can be guaranteed.

As can be seen from Fig. 5, compared with the corresponding embodiment of Fig. 2, the flowchart 500 of the method for pushing information in the present embodiment reflects the steps of verifying prescription information, and pushing abnormal prompt information if the prescription information fails to pass the verification. Therefore, the solution described in the present embodiment can guarantee the authenticity and validity of the prescription, and can avoid the abuse of the prescription on the Internet platforms.

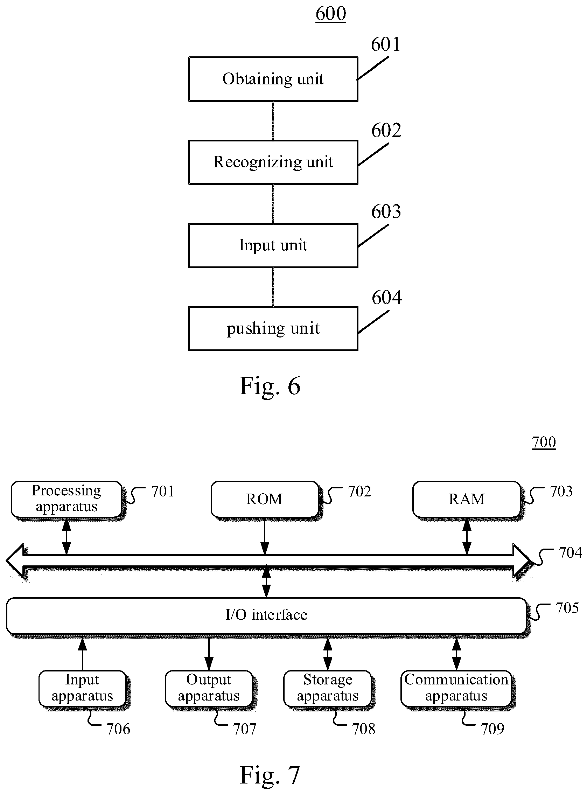

Further referring to Fig. 6, as an implementation of the method shown in the above figures, an embodiment of the present disclosure provides an apparatus for pushing information. The embodiment of the apparatus corresponds to the embodiment of the method shown in Fig. 2. The apparatus may be specifically applied to various electronic devices.

As shown in Fig. 6, the apparatus 600 for pushing information of the present embodiment includes: an obtaining unit 601, a recognizing unit 602, an input unit 603, and a pushing unit 604. The obtaining unit 601 is configured to obtain a prescription image presenting prescription information; the recognizing unit 602 is configured to recognize the prescription image to obtain a recognition result; the input unit 603 is configured to input the recognition result into a pre-trained prescription image analyzing model to obtain the prescription information presented in the prescription image; and the pushing unit 604 is configured to push information based on the prescription information.

Step 201, step 202, step 203, and step 204 in the corresponding embodiment of Fig. 2 may be referred to the specific processing of the acquiring unit 601, the recognizing unit 602, the input unit 603, and the pushing unit 604 of the apparatus 600 for pushing information in the present embodiment.

In some alternative implementations of the present embodiment, the pushing unit 604 may push information based on the prescription information, which includes the following steps: the pushing unit 604 may verify the prescription information, e.g., may verify the authenticity and validity of the prescription information, and then may push the information based on the verification result. As an example, if the verification result indicates that a prescription indicated by the prescription image is real and valid, the pushing unit 604 may push drugstore information of the selected drugstore.

In some alternative implementations of the present embodiment, the pushing unit 604 may push the information based on the verification result, which includes the following steps: if the verification result is determined to indicate that the prescription information passes the verification, the pushing unit 604 may push the drug purchase information based on the drug information in the prescription information. Specifically, the drug information may include a drug name, a drug specification, and a drug amount. The pushing unit 604 may match the drug name and the drug specification with information of each drug in a preset drug set, to find a drug indicated in the prescription image as a target drug; then may search at least one drugstore whose inventory amount of the target drug is greater than or equal to the drug amount; then may, for each drugstore among the found at least one drugstore, generate drug purchase information that includes the drug name, a drug brand, the drug specification, and a drugstore identifier (drugstore name) of the drugstore; and finally may push the generated at least one piece of drug purchase information. It should be noted that each piece of drug purchase information among the at least one piece of drug purchase information generally corresponds to a drugstore. Here, the drug purchase information may include a drug purchase link, and a user may usually click a drug purchase link presented on a user terminal, thereby purchasing a drug indicated by the prescription image.

In some alternative implementations of the present embodiment, the pushing unit 604 may push the information based on the verification result which includes the following steps: if the verification result is determined to indicate that the prescription information fails to pass the verification, the pushing unit 604 may push abnormal prompt information. The abnormal prompt information may be used to indicate that the prescription information is abnormal. As an example, the abnormal prompt information may be "your prescription is abnormal. Please re-upload a prescription image."

In some alternative implementations of the present embodiment, the pushing unit 604 may push the drug purchase information based on the drug information in the prescription information as follows. The pushing unit 604 may first obtain positioning information of a user terminal, where the prescription image is obtained from the user terminal, and then may determine at least one drugstore within a preset range based on the positioning information. The preset range may be a given city, i.e., the drugstore and a user uploading the prescription image are in the given city; or the preset range may be a preset distance, for example, two kilometers. Then, the pushing unit 604 may select a target drugstore from the at least one drugstore based on a preset credit rating and a position of the at least one drugstore, and an inventory amount and a price of a drug indicated by the drug information in the drugstore. Specifically, the credit rating may be represented as a star, which may be generated based on user evaluations, and may include five stars, four stars, three stars, two stars, and one star. The pushing unit 604 may first select drugstores from the at least one drugstore whose inventory amount of the drug indicated by the drug information is greater than or equal to the drug amount in the drug information as first drugstores; then may select, in an ascending order of sale prices of the drug indicated by the drug information, a preset first number of drugstores from the first drugstores as second drugstores; then may determine distances between the user terminal and the second drugstores based on the positioning information of the user terminal and positions of the second drugstores, to select, in an ascending order of distances, a preset second number of drugstores from the second drugstores as third drugstores; and finally may select a drugstore with a highest credit rating from the third drugstores as the target drugstore. The pushing unit 604 may push drug purchase information including a drugstore identifier of the target drugstore to the user terminal, where the prescription image is obtained from the user terminal. In this way, the target drugstore may be selected based on a plurality of dimensions, and the drugstore name of the target drugstore may be pushed to the user, thereby improving the abundance of the drug purchase information.

In some alternative implementations of the present embodiment, the drug information in the prescription information may include a drug price, i.e., a prescription may include a drug price of the drug. Then, the pushing unit 604 may select the target drugstore from the at least one drugstore based on the preset credit rating and the position of the at least one drugstore, and based on the inventory amount and the price of the drug indicated by the drug information in the drugstore, as follows. The pushing unit 604 may find, for each drugstore among the at least one drugstore, a preset weight corresponding to the preset credit rating of the drugstore as a first weight. Here, the pushing unit 604 may store a corresponding relationship between the credit rating and the preset weight. For example, a weight of a five-star drugstore may be 1, a weight of a four-star drugstore may be 0.8, and a weight of a three-star drugstore may be 0.6. Under normal conditions, the lower the credit rating is, the smaller the weight is. Then, the pushing unit 604 may determine a ratio of the drug price in the prescription to a price of the drug indicated by the drug information in the drugstore as a second weight. As an example, if the drug price in the prescription is CNY 36, and the price of the drug in the drugstore is CNY 25, the second weight may be 1.44. Here, an upper limit of the second weight may be set, for example, as 2. It should be noted that if the prescription includes at least two drug names, the pushing unit 604 may determine a ratio of a drug price of each drug to a price of the drug in the drugstore respectively, and may determine a largest ratio as the second weight. Then, the pushing unit 604 may find a preset weight corresponding to the inventory amount of the drug indicated by the drug information in the drugstore as a third weight. Here, if the inventory amount is greater than or equal to the drug amount in the prescription, the third weight may be 1; while if the inventory amount is less than the drug amount in the prescription, the third weight may be 0. Then, the pushing unit 604 may determine a distance between the drugstore and a user using the user terminal based on the positioning information of the user terminal and a position of the drugstore, and determine a fourth weight based on the distance. Specifically, the pushing unit 604 may store a corresponding relationship table of a corresponding relationship between the preset distance and the fourth weight, and the pushing unit 604 may search for the fourth weight corresponding to the determined distance from the corresponding relationship table. Then, the pushing unit 604 may determine a product of the first weight, the second weight, the third weight, and the fourth weight as a score of the drugstore. Finally, the pushing unit 604 may select the target drugstore from the at least one drugstore based on a score of the at least one drugstore. As an example, a drugstore with a highest score may be selected from the at least one drugstore as the target drugstore.

In some alternative implementations of the present embodiment, the pushing unit 604 may determine the fourth weight based on the distance, as follows. The pushing unit 604 may find a preset delivery duration corresponding to the distance. The pushing unit 604 may store a corresponding relationship table of a corresponding relationship between the preset distance and the delivery duration, and may search a delivery duration corresponding to the distance from the corresponding relationship table. For example, a delivery duration corresponding to a distance of 500 kilometers may be found to be 2 days. Here, the delivery duration is usually measured in days. Then, a ratio of a preset value to the delivery duration may be determined as the fourth weight. The preset value may be 1. As an example, if the delivery duration is 2 days, the fourth weight may be 0.5. Here, an upper limit of the fourth weight may be set, for example, as 2.

In some alternative implementations of the present embodiment, the pushing unit 604 may verify the prescription information as follows. The pushing unit 604 may determine whether the prescription information includes a target text. The target text is generally a preset text recorded in a normal prescription, and generally may include a patient name, a patient age, date, a drug name, a drug specification, and a drug amount. If the prescription information is determined not to include any text in the target text, the pushing unit 604 may push first abnormal prompt information. Here, the first abnormal prompt information may be used for indicating that a prescription indicated by the prescription image is false. As an example, the first abnormal prompt information may be "your prescription is false. Please re-upload a prescription image."

In some alternative implementations of the present embodiment, the pushing unit 604 may verify the prescription information as follows. The pushing unit 604 may determine whether the prescription information does not include all target texts in a target text set or does not include corresponding texts of all target texts. The target text is generally a preset text to be recorded in a normal prescription, and generally may include a patient name, a patient age, date, a drug name, a drug specification, and a drug amount. The corresponding text of the target text is usually a value of the target text. For example, if the target text is a patient name, the corresponding text may be Mr. A; while if the target text is a drug name, the corresponding text may be Banlangen granule. If the prescription information is determined not to include all target texts in the target text set or does not include the corresponding texts of all target texts, the pushing unit 604 may push second abnormal prompt information. Here, the second abnormal prompt information may be used for indicating that a prescription indicated by the prescription image is incomplete. As an example, the second abnormal prompt information may be "your prescription is incomplete. Please re-upload a prescription image."

In some alternative implementations of the present embodiment, the pushing unit 604 may verify the prescription information as follows. The pushing unit 604 may extract time-related information from the prescription information. The time-related information may include prescription issue time and a prescription validity duration. As an example, if the prescription is annotated with that: the prescription is valid on the current day, the prescription validity duration being less than one day may be extracted; while if the prescription is annotated with that: the prescription is valid within three days, the prescription validity duration being three days may be extracted. The pushing unit 604 may determine a time difference between current time and the prescription issue time, and determine whether the time difference is greater than the prescription validity duration. As an example, if the current time is December 30,2019, the prescription issue time is December 26, 2019, and the prescription validity duration is three days, the time difference between the current time of December 30, 2019 and the prescription issue time of December 26 2019 may be determined to be four days, which is greater than the prescription validity duration of three days. If the time difference is determined to be greater than the prescription validity duration, the pushing unit 604 may push third abnormal prompt information. Here, the third abnormal prompt information may be used for indicating that a prescription indicated by the prescription image is expired. As an example, the third abnormal prompt information may be "your prescription is expired."

In some alternative implementations of the present embodiment, the pushing unit 604 may verify the prescription information through the following steps: the pushing unit 604 may determine whether the prescription corresponding to the prescription information is present in a used prescription set. Here, the pushing unit 604 may store the used prescription set, and prescriptions stored in the used prescription set are usually prescriptions that have been used by a user to buy a drug. If the prescription corresponding to the prescription information is present in the employed prescription set, the pushing unit 604 may push fourth abnormal prompt information. Here, the fourth abnormal prompt information may be used for indicating that a prescription indicated by the prescription image is used. As an example, the fourth abnormal prompt information may be "your prescription is employed."

In some alternative implementations of the present embodiment, the recognition result may include position information used to indicate a position of the character in the prescription image. A direction of the character may be determined based on a position of each character in the prescription image. Here, the position information may be represented using coordinates in a preset coordinate system. The coordinate system may use any point of the prescription image (for example, a vertex of the prescription image) as the origin of coordinates, and use straight lines parallel to two adjoining sides of the prescription image as the abscissa axis and the ordinate axis of the coordinate system. The prescription image analyzing model may be obtained by training, which includes the following steps: a training sample set may be first obtained. A training sample in the training sample set usually include a sample recognition result and sample prescription information. The sample recognition result is generally recognized from the sample prescription image. The sample prescription information is generally determined based on the position of each character in the corresponding sample recognition result in the sample prescription image. Specifically, a feature text may be selected from the sample prescription image. The feature text may be manually annotated by the pharmacist, for example, may include hospital, clinic, health station, name, age, gender, Rp, doctor, physician, and date. Then, the sample prescription information may be obtained based on analysis of the feature text. For each feature text among a plurality of feature texts, coordinates of the feature text (for example, coordinates of a first character) may be used as a starting point, a character string with a closest distance from the feature text may be determined based on a vector of a text direction (character read or write direction of the feature text), and the character string may be determined as prescription information corresponding to the feature text. Then, the prescription image analyzing model may trained by taking sample recognition results of training samples in the training sample set as an input, and by taking sample prescription information corresponding to inputted sample recognition results as an expected output.

Referring to Fig. 7 below, Fig. 7 shows a schematic structural diagram of an electronic device 700 (e.g., the server in Fig. 1) adapted to implement embodiments of the present disclosure. The server shown in Fig. 7 is merely an example, and should not impose any limitation on the functions and scope of use of some embodiments of the present disclosure.

As shown in Fig. 7, the electronic device 700 may include a processing apparatus 701 (e.g., a central processing unit or a graphics processing unit), which may execute various appropriate actions and processes in accordance with a program stored in a read only memory (ROM) 702 or a program loaded into a random-access memory (RAM) 703 from a storage apparatus 708. The RAM 703 further stores various programs and data required by operations of the electronic device 700. The CPU 701, the ROM 702, and the RAM 703 are connected to each other through a bus 704. An input/output (I/O) interface 705 is also connected to the bus 704.

In general, the following apparatuses may be connected to the I/O interface 705: an input apparatus 706 including a touch screen, a touch pad, a keyboard, a mouse, a camera, a microphone, an accelerometer, a gyroscope, or the like; an output apparatus 707 including a liquid crystal display device (LCD), a speaker, a vibrator, or the like; a storage apparatus 708 including a magnetic tape, a hard disk, or the like; and a communication apparatus 709. The communication apparatus 709 may allow the electronic device 700 to exchange data with other devices through wireless or wired communication. While Fig. 7 shows the electronic device 700 having various apparatuses, it should be understood that it is not necessary to implement or provide all of the apparatuses shown in the figure. More or fewer apparatuses may be alternatively implemented or provided. Each block shown in Fig. 7 may represent an apparatus, or represent a plurality of apparatuses as required.

In particular, according to the embodiments of the present disclosure, the process described above with reference to the flow chart may be implemented as a computer software program. For example, the embodiments of the present disclosure include a computer program product, which includes a computer program that is tangibly embedded in a computer readable medium. The computer program includes a program code for executing the method as shown in the flow chart. In such an embodiment, the computer program may be downloaded and installed from a network via the communication apparatus 709, or be installed from the storage apparatus 708, or be installed from the ROM 702. The computer program, when executed by the processing apparatus 701, implements the above functions as defined by the method of the embodiments of the present disclosure. It should be noted that the computer readable medium of the embodiments of the present disclosure may be a computer readable signal medium or a computer readable storage medium, or any combination of the above two. An example of the computer readable storage medium may include, but is not limited to: electric, magnetic, optical, electromagnetic, infrared, or semiconductor systems, apparatuses, elements, or a combination of any of the above. A more specific example of the computer readable storage medium may include, but is not limited to: an electrical connection with one or more pieces of wire, a portable computer disk, a hard disk, a random-access memory (RAM), a read only memory (ROM), an erasable programmable read-only memory (EPROM or flash memory), an optical fiber, a portable compact disk read-only memory (CD-ROM), an optical memory, a magnetic memory, or any suitable combination of the above. In the embodiments of the present disclosure, the computer readable storage medium may be any tangible medium containing or storing programs which may be used by, or used in combination with, a command execution system, apparatus or element. In the embodiments of the present disclosure, the computer readable signal medium may include a data signal in the base band or propagating as a part of a carrier wave, in which a computer readable program code is carried. The propagating data signal may take various forms, including but not limited to an electromagnetic signal, an optical signal, or any suitable combination of the above. The computer readable signal medium may also be any computer readable medium except for the computer readable storage medium. The computer readable signal medium is capable of transmitting, propagating or transferring programs for use by, or use in combination with, a command execution system, apparatus or element. The program code contained on the computer readable medium may be transmitted with any suitable medium, including but not limited to: wire, an optical cable, a RF (radio frequency) medium etc., or any suitable combination of the above.

The computer readable medium may be included in the above electronic device; or may be a stand-alone computer readable medium without being assembled into the electronic device. The computer readable medium carries one or more programs, where the one or more programs, when executed by the electronic device, cause the electronic device to: obtain a prescription image presenting prescription information; recognize the prescription image to obtain a recognition result; input the recognition result into a pre-trained prescription image analyzing model to obtain the prescription information presented in the prescription image; and push information based on the prescription information.

A computer program code for executing operations in the embodiments of the present disclosure may be compiled using one or more programming languages or combinations thereof. The programming languages include object-oriented programming languages, such as Java, Smalltalk or C++, and also include conventional procedural programming languages, such as "C" language or similar programming languages. The program code may be completely executed on a user's computer, partially executed on a user's computer, executed as a separate software package, partially executed on a user's computer and partially executed on a remote computer, or completely executed on a remote computer or server. In the case where a remote computer is involved, the remote computer may be connected to a user computer through any kind of networks, including a local area network (LAN) or a wide area network (WAN), or may be connected to an external computer (for example, connected through the Internet using an Internet service provider).

The flow charts and block diagrams in the figures illustrate architectures, functions and operations that may be implemented according to the systems, methods and computer program products of the various embodiments of the present disclosure. In this regard, each of the blocks in the flow charts or block diagrams may represent a module, a program segment, or a code portion, said module, program segment, or code portion including one or more executable instructions for implementing specified logic functions. It should also be noted that, in some alternative implementations, functions annotated in the blocks may also occur in an order different from the order annotated in the figures. For example, any two blocks presented in succession may be executed substantially in parallel, or sometimes be executed in a reverse sequence, depending on the functions involved. It should also be noted that each block in the block diagrams and/or flow charts as well as a combination of blocks in the block diagrams and/or flow charts may be implemented using a dedicated hardware-based system executing specified functions or operations, or by a combination of dedicated hardware and computer instructions.

The units involved in the embodiments of the present disclosure may be implemented by software, or may be implemented by hardware. The described units may also be provided in a processor, for example, described as: a processor including an acquiring unit, a recognizing unit, an input unit, and a pushing unit. The names of the units do not constitute a limitation to such units themselves in some cases. For example, the pushing unit may be further described as "a unit configured to perform information push based on prescription information."

The above description only provides explanation of the preferred embodiments and the employed technical principles of the present disclosure. It should be appreciated by those skilled in the art that the inventive scope involved in embodiments of the present disclosure is not limited to the technical solutions formed by the particular combinations of the above technical features. The inventive scope should also cover other technical solutions formed by any combination of the above technical features or equivalent features thereof without departing from the inventive concept of the present disclosure, for example, the technical solutions formed by interchanging the above features with, but not limited to, technical features with similar functions disclosed in the embodiments of the present disclosure.

## Claims

1. A method for pushing information, comprising:
obtaining a prescription image presenting prescription information;
recognizing the prescription image to obtain a recognition result;
inputting the recognition result into a pre-trained prescription image analyzing model to obtain the prescription information presented in the prescription image; and
pushing information based on the prescription information.

2. The method according to claim 1, wherein the pushing information based on the prescription information comprises:
verifying the prescription information; and
pushing the information based on a verification result.

3. The method according to claim 2, wherein the pushing the information based on the verification result comprises:
pushing drug purchase information based on drug information in the prescription information when the verification result indicates that the prescription information passes a verification.

4. The method according to claim 2, wherein the pushing the information based on the verification result comprises:
pushing abnormal prompt information when the verification result indicates that the prescription information fails to pass the verification.

5. The method according to claim 3, wherein the pushing the drug purchase information based on the drug information in the prescription information comprises:
obtaining positioning information of a user terminal, the prescription image being obtained from the user terminal;
determining at least one drugstore within a preset range based on the positioning information;
selecting a target drugstore from the at least one drugstore based on a preset credit rating and a position of the at least one drugstore, and based on an inventory amount and a price of a drug indicated by the drug information in the at least one drugstore; and
pushing the drug purchase information, wherein the drug purchase information comprises a drugstore identifier of the target drugstore.

6. The method according to claim 5, wherein the drug information comprises a drug price; and
the selecting the target drugstore from the at least one drugstore based on the preset credit rating and the position of the at least one drugstore, and based on the inventory amount and the price of the drug indicated by the drug information in the at least one drugstore comprises:
for each drugstore of the at least one drugstore: finding a preset weight corresponding to a preset credit rating of the drugstore as a first weight, determining a ratio of the drug price to the price of the drug indicated by the drug information in the drugstore as a second weight, finding a preset weight corresponding to the inventory amount of the drug indicated by the drug information in the drugstore as a third weight, determining a distance between the drugstore and a user using the user terminal based on the positioning information of the user terminal and a position of the drugstore, determining a fourth weight based on the distance, and determining a product of the first weight, the second weight, the third weight, and the fourth weight as a score of the drugstore; and
selecting the target drugstore from the at least one drugstore based on the score of each drugstore.

7. The method according to claim 6, wherein the determining the fourth weight based on the distance comprises:
finding a preset delivery duration corresponding to the distance, and determining a ratio of a preset value to the delivery duration as the fourth weight.

8. The method according to claim 4, wherein the verifying the prescription information comprises:
determining whether the prescription information comprises a target text; and
the pushing the abnormal prompt information when the verification result indicates that the prescription information fails to pass the verification comprises:
pushing first abnormal prompt information when determining that the prescription information does not comprise the target text, wherein the first abnormal prompt information is configured to indicate that a prescription indicated by the prescription image is false.

9. The method according to claim 4, wherein the verifying the prescription information comprises:
determining whether the prescription information does not comprise a target text in a target text set or a corresponding text of the target text; and
the pushing the abnormal prompt information when the verifying result indicates that the prescription information fails to pass the verification comprises:
pushing second abnormal prompt information when determining that the prescription information does not comprise the target text in the target text set or does not comprise the corresponding text of the target text, wherein the second abnormal prompt information is configured to indicate that a prescription indicated by the prescription image is incomplete.

10. The method according to claim 4, wherein the verifying the prescription information comprises:
extracting time-related information from the prescription information, wherein the time-related information comprises a prescription issuing time and a prescription validity duration;
determining a time difference between a current time and the prescription issuing time, and determining whether the time difference is greater than the prescription validity duration; and
the pushing the abnormal prompt information when the verifying result indicates that the prescription information fails to pass the verification comprises:
pushing third abnormal prompt information when determining that the time difference is greater than the prescription validity duration, wherein the third abnormal prompt information is configured to indicate that a prescription indicated by the prescription image is expired.

11. The method according to claim 4, wherein the verifying the prescription information comprises:
determining whether a prescription corresponding to the prescription information is present in a used prescription set; and
the pushing the abnormal prompt information when the verifying result indicates that the prescription information fails to pass the verification comprises:
pushing fourth abnormal prompt information when determining that the prescription corresponding to the prescription information is present in the used prescription set, wherein the fourth abnormal prompt information is configured to indicate that a prescription indicated by the prescription image is used.

12. The method according to any one of claims 1-11, wherein the recognition result comprises position information configured to indicate a position of a character in the prescription image; and
wherein the prescription image analyzing model is obtained by training comprising:
obtaining a training sample set, wherein a training sample includes a sample recognition result and sample prescription information, the sample recognition result is recognized from a sample prescription image, and the sample prescription information is determined based on a position of each character of a corresponding sample recognition result in the sample prescription image; and
training the prescription image analyzing model by taking sample recognition results of training samples in the training sample set as an input, and by taking sample prescription information corresponding to inputted sample recognition results as an expected output.

13. An apparatus for pushing information, comprising:
an obtaining unit configured to obtain a prescription image presenting prescription information;
a recognizing unit configured to recognize the prescription image to obtain a recognition result;
an input unit configured to input the recognition result into a pre-trained prescription image analyzing model to obtain the prescription information presented in the prescription image; and
a pushing unit configured to push information based on the prescription information.

14. An electronic device, comprising:
one or more processors; and
a storage apparatus storing one or more programs thereon,
the one or more programs, when executed by the one or more processors, cause the one or more processors to implement the method according to any one of claims 1 to 12.

15. A computer readable medium, storing a computer program thereon, wherein the computer program, when executed by a processor, implements the method according to any one of claims 1 to 12.
